# FASCICULE DE BREVET EUROPEEN

(11) **EP 1 048 322 B1**
(45) Date de publication et mention de la délivrance du brevet: **21.09.2005**
(21) Numéro de dépôt: 00401148.2
(22) Date de dépôt: 26.04.2000
(51) Int. Cl.: A61N 1/368

(54) **Dispositif médical implantable actif, notamment stimulateur cardiaque, défibrillateur et/ou cardioverteur de type à commutation automatique de mode DDD/AAI perfectionné**
Implantierbarer Herzschrittmacher, Defibrillator und/oder Kardiovertierer mit automatischer Betriebsartumschaltung DDD/AAI
Implantable pacemaker, defibrillator and/or cardioverter with improved automatic mode switching DDD/AAI

(30) Priorité: 26.04.1999 FR 9905230
(43) Date de publication de la demande: 02.11.2000
(73) Titulaire: ELA MEDICAL (Société anonyme), 92541 Montrouge (FR)
(72) Inventeur: Bonhour, Anne, 92410 Ville d'Avray (FR); Bonnet, Jean-Luc, 92190 Montrouge (FR)
(74) Mandataire: Dupuis-Latour, Dominique

(56) Documents cités:
- EP-A- 0 488 904
- WO-A-99/10044
- US-A- 5 683 426

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, dispositifs "multisite" (triple ou quadruple chambre), défibrillateurs et/ou cardioverteurs, permettant de délivrer au coeur des impulsions électriques de faible énergie pour le traitement des troubles du rythme cardiaque.

Elle concerne plus particulièrement ceux de ces dispositifs qui comprennent des circuits de stimulation à commutation automatique de mode de fonctionnement (CAM), comme décrit par exemple par le EP-A-0 488 904 (ELA Médical).

Plus précisément ces dispositifs, qui comportent des moyens de stimulation et de détection à la fois sur l'oreillette et le ventricule, peuvent opérer selon deux modes, DDD ou AAI (le mode AAI étant en fait un mode DDD comprenant un délai atrio-ventriculaire allongé), avec basculement automatique d'un mode à l'autre.

Le mode de fonctionnement de base est un mode AAI, avec une stimulation auriculaire simple chambre. Ce mode est maintenu tant que la conduction atrio-ventriculaire est normale, c'est-à-dire tant que chaque événement auriculaire (détection auriculaire, correspondant à une activité spontanée, ou stimulation auriculaire) est suivi d'une détection ventriculaire associée.

Dans certaines circonstances, par exemple des épisodes d'effort, peuvent cependant apparaître des blocs atrio-ventriculaires (BAV), dits "paroxystiques", entraînant un défaut de dépolarisation du ventricule (les BAV paroxystiques peuvent cependant intervenir dans d'autres circonstances que celles d'un effort). Dans ce cas, le stimulateur bascule automatiquement en mode DDD automatique, avec des paramètres optimisés pour cette situation de BAV temporaire. En particulier, pour favoriser le retour d'une conduction atrio-ventriculaire spontanée, le stimulateur applique un délai atrio-ventriculaire (DAV) relativement long, susceptible de laisser s'exprimer la conduction atrio-ventriculaire du patient.

Après disparition du BAV, et donc rétablissement de la conduction AV (atrio-ventriculaire) spontanée, le stimulateur retourne automatiquement au mode AAI, dès lors qu'un certain nombre de conditions correspondantes sont remplies.

Le point de départ de l'invention est la constatation que ce mode opératoire n'est pas bien adapté à tous les types de BAV.

il existe certes d'autres types de dispositifs, comme décrit par exemple par le US-A-5 683 426, détectant la survenue d'un BAV et étudiant son évolution dans le temps. Mais, ici encore, l'action prise sur détection de BAV est la même quelle que soit le type de BAV, paroxystique ou non.

Plus précisément, le basculement au mode DDD avec un DAV relativement long, qui cherche à favoriser la conduction AV spontanée, est bien adapté aux BAV dits "paroxystiques", c'est-à-dire déclenchés par un effort ou autre cause externe, mais de caractère temporaire.

En revanche, lorsqu'un patient évolue en BAV chronique, c'est-à-dire sur une période de plusieurs jours, il se retrouvera stimulé en permanence avec un DAV long.

Or cet allongement du DAV n'est pas nécessaire, car il est inutile de chercher à favoriser une conduction AV spontanée qui ne s'exprimera plus jamais, ou presque plus jamais ; bien plus, cet allongement du DAV est même néfaste car, en stimulation permanente, les DAV optimaux sont généralement plus courts que les intervalles PR spontanés des patients (intervalles compris entre une détection auriculaire P et une détection ventriculaire R).

L'invention propose de remédier à cette difficulté, en apportant un perfectionnement aux dispositifs connus.

Le type de dispositif auquel s'applique l'invention est un dispositif à commutation de mode DDD/AAI tel que décrit par exemple dans le EP-A-0 488 904 précité, c'est-à-dire comprenant : des moyens de recueil des événements auriculaires et ventriculaires spontanés ; des moyens de détection d'un bloc atrio-ventriculaire empêchant la conduction spontanée atrio-ventriculaire ; et des moyens de stimulation ventriculaire et auriculaire, la stimulation ventriculaire étant appliquée sur détection d'un bloc atrio-ventriculaire, après écoulement d'un délai atrio-ventriculaire programmé déclenché sur un événement auriculaire, spontané ou stimulé. Selon l'invention, ce dispositif comprend en outre des moyens aptes à discriminer le caractère paroxystique ou chronique des blocs atrio-ventriculaires.

Il comprend en outre, avantageusement, des moyens aptes, en cas de bloc atrio-ventriculaire chronique avéré, à désactiver la commutation automatique de mode du dispositif et à basculer celui-ci en mode DDD. Avantageusement, ces moyens sont également aptes à reprogrammer le délai atrio-ventriculaire, notamment à une valeur plus courte que la valeur antérieurement programmée.

Très avantageusement, les moyens de discrimination comprennent des moyens aptes, dans une première phase, à suspecter un bloc atrio-ventriculaire chronique et des moyens aptes, dans une seconde phase, à confirmer la présence d'un bloc atrio-ventriculaire chronique en cas de suspicions consécutives de bloc atrio-ventriculaire chronique pendant une durée prédéterminée.

La première phase peut notamment être mise en oeuvre sur un intervalle journalier et la seconde phase est mise en oeuvre sur un intervalle pluri-journalier, avantageusement un intervalle de trois jours.

Un critère de suspicion de bloc atrio-ventriculaire chronique peut être l'absence de conduction spontanée pendant ladite durée prédéterminée, ou encore l'absence de conduction spontanée après un nombre prédéterminé de recherches de conduction spontanée, par exemple environ 500 recherches sur un intervalle journalier.

Un critère de suspicion de bloc atrio-ventriculaire chronique peut également être l'application, pendant ladite durée prédéterminée, d'une stimulation ventriculaire sur un nombre ou un pourcentage de cycles cardiaques supérieur à une valeur prédéterminée, par exemple supérieur à 75 %, avec un délai atrio-ventriculaire de durée supérieure à une valeur prédéterminée, par exemple supérieure à 200 ms.

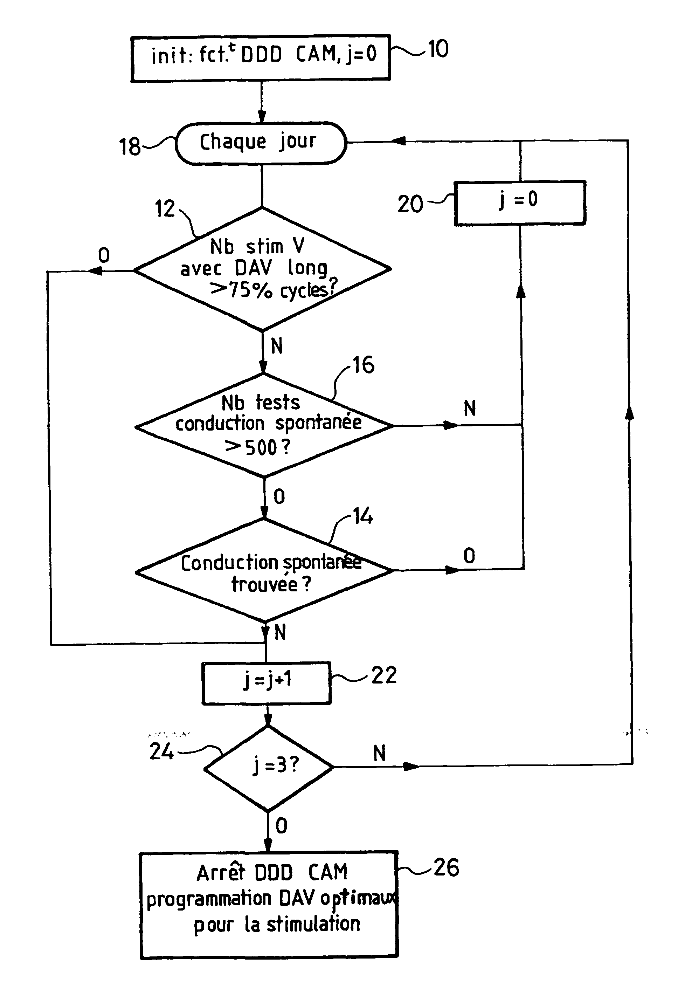

On va maintenant décrire un exemple de mise en oeuvre de l'invention en référence à la figure annexée, qui est un organigramme général de l'algorithme permettant de mettre en oeuvre les fonctions de l'invention.

L'invention est avantageusement mise en oeuvre par un logiciel intégré à l'algorithme DDD CAM d'un stimulateur cardiaque de type classique. Initialement, le stimulateur, qui a détecté des BAV, opère en mode DDD (étape 10).

Les fonctions mises en oeuvre par le logiciel de l'invention opèrent d'abord un diagnostic de BAV chronique, ce diagnostic étant réalisé en deux étapes(un BAV chronique est défini comme un bloc de conduction de haut degré ou un BAV 1).

La première étape consiste à établir une "suspicion" de BAV chronique, c'est-à-dire à déterminer la présence de BAV dont les caractéristiques vont au-delà de simples BAV paroxystiques.

L'algorithme suspecte l'apparition d'un BAV chronique si :
- le ventricule a été stimulé avec un DAV long, supérieur à 200 ms (par exemple), sur plus de 75 % (par exemple) des cycles cardiaques durant les dernières 24 heures (étape 12) - cette situation clinique étant typiquement celle que l'on veut éviter de faire perdurer avec le DDD CAM,
   ou si
- aucune conduction spontanée n'a été trouvée (étape 14), cette conduction ayant été recherchée (étape 16) au moins 500 fois (par exemple). Cette recherche est opérée par augmentation du DAV par l'algorithme DDD CAM, comme décrit dans le EP-A-0 488 904 précité, et la valeur de 500 recherches correspond approximativement à une recherche opérée au moins la moitié du temps sur une journée, compte tenu des périodes sans recherche.

Cette première phase de suspicion de diagnostic est opérée par analyse du fonctionnement du stimulateur sur une durée typique de 24 heures (étape 18) et réinitialisée (étape 20) si l'un des critères de suspicion n'est pas ou plus vérifié.

Bien entendu, les divers paramètres précités donnés à titre d'exemple (24 heures, 75 % des cycles, 200 ms et 500 fois) sont des valeurs programmables, ajustables en fonction du patient et de l'optimisation du diagnostic sur le plan clinique.

La seconde phase consiste à confirmer le diagnostic de BAV chronique, le dispositif considérant par exemple qu'il y a BAV chronique avéré en cas de suspicion de BAV chronique pendant trois jours consécutifs (étapes 22 et 24) ; dans la négative, l'algorithme est réinitialisé (retour à l'étape 18). L'action décidée par le dispositif en cas de BAV chronique avéré consiste (étape 26) :
- d'une part, à désactiver la commutation automatique de mode du stimulateur, c'est-à-dire à passer du mode DDD CAM au mode DDD simple, le DAV n'étant alors plus monitoré en fonction de la conduction spontanée - qui ne s'exprime plus - du patient, et
- d'autre part, à reprogrammer le DAV (durée et hystérésis) à des valeurs optimisées pour la stimulation permanente, plus courtes, correspondant généralement à des valeurs préprogrammées du dispositif.

## Revendications

1. Un dispositif médical implantable actif, notamment un stimulateur cardiaque, défibrillateur et/ou cardioverteur de type à commutation automatique de mode DDD/AAI, comprenant :
- des moyens de recueil des événements auriculaires et ventriculaires spontanés,
- des moyens de détection d'un bloc atrio-ventriculaire empêchant la conduction spontanée atrio-ventriculaire, et
- des moyens de stimulation ventriculaire et auriculaire, la stimulation ventriculaire étant appliquée sur détection d'un bloc atrio-ventriculaire, après écoulement d'un délai atrio-ventriculaire programmé déclenché sur un événement auriculaire, spontané ou stimulé,
dispositif **caractérisé en ce qu'**il comprend en outre des moyens aptes à discriminer le caractère paroxystique ou chronique des blocs atrio-ventriculaires.

2. Le dispositif de la revendication 1, comprenant en outre des moyens aptes, en cas de bloc atrio-ventriculaire chronique avéré, à désactiver la commutation automatique de mode du dispositif et à basculer celui-ci en mode DDD.

3. Le dispositif de la revendication 2, comprenant en outre des moyens aptes, en cas de bloc atrio-ventriculaire chronique avéré, à reprogrammer le délai atrio-ventriculaire.

4. Le dispositif de la revendication 3, dans lequel le délai atrio-ventriculaire est reprogrammé à une valeur plus courte que la valeur antérieurement programmée.

5. Le dispositif de la revendication 1, dans lequel les moyens de discrimination comprennent :
- des moyens aptes, dans une première phase, à suspecter un bloc atrio-ventriculaire chronique et,
- des moyens aptes, dans une seconde phase, à confirmer la présence d'un bloc atrio-ventriculaire chronique en cas de suspicions consécutives de bloc atrio-ventriculaire chronique pendant une durée prédéterminée.

6. Le dispositif de la revendication 5, dans lequel la première phase est mise en oeuvre sur un intervalle journalier et la seconde phase est mise en oeuvre sur un intervalle pluri-journalier, avantageusement un intervalle de trois jours.

7. Le dispositif de la revendication 5, dans lequel l'un au moins des critères de suspicion de bloc atrio-ventriculaire chronique est l'absence de conduction spontanée pendant ladite durée prédéterminée.

8. Le dispositif de la revendication 5 ou 7 dans lequel l'un au moins des critères de suspicion de bloc atrio-ventriculaire chronique est l'absence de conduction spontanée après un nombre prédéterminé de recherches de conduction spontanée.

9. Le dispositif de la revendication 8 dans lequel ledit nombre prédéterminé est d'environ 500 recherches sur un intervalle journalier.

10. Le dispositif de la revendication 5, dans lequel l'un au moins des critères de suspicion de bloc atrio-ventriculaire chronique est l'application, pendant ladite durée prédéterminée, d'une stimulation ventriculaire sur un nombre ou un pourcentage de cycles cardiaques supérieur à une valeur prédéterminée avec un délai atrio-ventriculaire de durée supérieure à une valeur prédéterminée.

11. Le dispositif de la revendication 10, dans lequel ladite valeur prédéterminée est de 75 % et ladite valeur prédéterminée est 200 ms.

## Patentansprüche

1. Aktive implantierbare medizinische Vorrichtung, insbesondere Herzschrittmacher, Defibrillator und/oder Kardioverter vom Typ mit automatischer Kommutation vom Modus DDD/AAI, umfassend:
- Mittel zum Sammeln von spontanen Herzvorhof- und ventrikulären Ereiguissen,
- Mittel zur Erkennung eines atrio-ventrikulären Blocks, welche die atrio-ventrikuläre spontane Leitung behindern, und
- Mittel zur ventrikulären und Herzvorhofstimulation, wobei die ventrikuläre Stimulation angewendet wird auf Erkennung eines atrio-ventrikulären Blocks, nach Verlaufen einer programmierten atrio-ventrikulären Verzögerung, ausgelöst auf ein spontanes oder stimuliertes Herzvorhofereignis,
wobei die Vorrichtung **dadurch gekennzeichnet ist, dass** sie weiterhin Mittel umfasst, die eingerichtet sind, den paroxystischen oder chronischen Charakter von atrio-ventrikulären Blöcken zu unterscheiden.

2. Vorrichtung nach Anspruch 1, weiterhin umfassend Mittel, die eingerichtet sind, im Falle eines erwiesenen chronischen atrio-ventrikulären Blocks, die automatische Kommutation des Modus der Vorrichtung zu deaktivieren, und diese in Modus DDD zu schalten.

3. Vorrichtung nach Anspruch 2, weiterhin umfassend Mittel, die eingereichtet sind, im Falle eines erwiesenen chronischen atrio-ventrikulären Blocks, die atrio-ventrikuläre Verzögerung umzuprogammieren.

4. Vorrichtung nach Anspruch 3, in welcher die atrio-ventrikuläre Verzögerung umprogrammiert wird auf einen kürzeren Wert als der zuvor programmierte.

5. Vorrichtung nach Anspruch 1, in welcher die Mittel zur Unterscheidung umfassen:
- Mittel, die eingerichtet sind, in einer ersten Phase, einen chronischen atrio-ventrikulären Block zu vermuten, und
- Mittel, die eingerichtet sind, in einer zweiten Phase, däs Vorhandensein eines chronischen atrio-ventrikulären Blocks zu bestätigen im Falle von aufeinander folgenden Vermutungen eines chronischen atrio-ventrikulären Blocks während einer vorherbestimmten Dauer.

6. Vorrichtung nach Anspruch 5, in welcher die erste Phase in einem täglichen Intervall angewandt wird und die zweite Phase in einem mehrtägigen Intervall angewandt wird, vorteilhafterweise ein Intervall von drei Tagen.

7. Vorrichtung nach Anspruch 5, in welcher wenigstens eines der Kriterien der Vermutung eines chronischen atrio-ventrikulären Blocks die Abwesenheit von spontaner Leitung während der vorbestimmten Dauer ist.

8. Vorrichtung nach Anspruch 5 oder 7, in welcher wenigstens eines der Kriterien der Vermutung eines chronischen atrio-ventrikulären Blocks die Abwesenheit der spontanen Leitung nach einer vorherbestimmten Anzahl von Suchen nach spontaner Leitung ist.

9. Vorrichtung nach Anspruch 8, in welcher die vorbestimmte Anzahl etwa 500 Suchen über ein tägliches Intervall ist.

10. Vorrichtung nach Anspruch 5, in welcher wenigstens eines der Kriterien der Vermutung eines chronischen atrio-ventrikulären Blocks die Anwendung, während der vorbestimmten Dauer, einer ventrikulären Stimulation über eine Anzahl oder einen Prozentsatz von Herzzyklen ist, größer als ein vorbestimmter Wert, mit einer atrio-ventrikulären Verzögerung von einer Dauer, die größer als ein vorbestimmter Wert ist.

11. Vorrichtung nach Anspruch 10, in welcher der vorbestimmte Wert 75% und der vorbestimmte Wert 200 ms ist.

## Claims

1. An active implantable medical device, in particular a cardiac pacemaker, defibrillator and/or cardioverter having an automatic DDDIAAI mode switching, which includes:
- means for sensing spontaneous atrial and ventricular events,
- means for detecting an atrio-ventricular block condition preventing spontaneous atria-ventricular conduction, and
- means for stimulating the atrium and the ventricle, the ventricular stimulation being applied upon detection of an atrio-ventricular block, after the completion of a programmed atrio-ventricular delay started on a spontaneous or stimulated atrial event,
said device being **characterised by** further including means for discriminating between a paroxystic and a chronic character of the atrio-ventricular blocks.

2. The device of claim 1, further including means for, in the event of a determined chronic atrio-ventricular block, deactivating the automatic mode switching of the device and switching the latter to a DDD operating mode.

3. The device of claim 2, further including means for, in the event of a determined chronic atrio-ventricular block, reprogramming the atrio-ventricular delay.

4. The device of claim 3, wherein the atrio-ventricular delay is reprogrammed to a value shorter than the previously programmed value.

5. The device of claim 1, wherein the means for discriminating include :
. means for, in a first phase, suspecting a chronic atrio-ventricular block condition, and
. means for, in second phase, confirming the presence of a chronic atrio-ventricular block in the event of consecutive suspicions of chronic atrio-ventricular blocks for a predetermined time.

6. The device of claim 5, wherein the first phase is implemented over a daily interval, and the second phase is implemented over a multi-day interval, preferably over a three-day interval.

7. The device of claim 5, wherein at least one of the criterions of suspicion of a chronic atrio-ventricular block is the absence of spontaneous conduction over said predetermined time.

8. The device of claim 5 or 7, wherein at least one of the criterions of suspicion of a chronic atrio-ventricular block is the absence of spontaneous conduction after a predetermined number of searches for spontaneous conductions.

9. The device of claim 8, wherein said predetermined number of searches is about 500 searches over a daily interval.

10. The device of claim 5, wherein at least one of the criterions of suspicion of a chronic atrio-ventricular block is the application, during said predetermined period, of a number, or a percentage, of cardiac cycles that is higher than a predetermined value, showing an atrio-ventricular delay of a duration greater than a predetermined value.

11. The device of claim 10, wherein said predetermined value is 75 % and said predetermined value is 200 ms.
